# EUROPEAN PATENT APPLICATION

(11) **EP 2 138 171 A1**
(43) Date of publication of application: **30.12.2009**
(21) Application number: 08425431.7
(22) Date of filing: 18.06.2008
(51) Int. Cl.: A61K 31/045, A61K 36/28, A61K 36/73, A61P 15/14, A61P 15/00, A61P 31/04

(54) **Veterinarian compositions containing terpinen-4-ol for the treatment and prevention of mastitis**

(71) Applicant: ISTITUTO SUPERIORE DI SANITA', 00161 Roma (IT); Institut National de la Recherche Agronomique, 75338 Paris Cedex 07 (FR); Istituto Zooprofilattico Sperimentale delle Regioni Lazio e Toscana, 00178 Roma-Capannelle (IT)
(72) Inventor: Aureli, Paolo, 00143 Roma (IT); Ferrini, Anna Maria, 00177 ROMA (IT); Montel, Marie Christine, 75338 PARIS Cedex 07 (FR); Amatiste, Simonetta, 00178 ROMA-CAPANNELLE (IT); Rosati, Remo, 00178 ROMA-CAPANNELLE (IT)
(74) Representative: Gitto, Serena

(57) **Abstract**

The present invention concerns therapeutically effective terpinen-4-ol based compositions with a broad spectrum of the activity against a wide range of mastitis causing bacteria of the genera *Staphylococcus* spp, *Streptococcus* spp, *Escherichia* sppp, *Enterococcus* spp, *Serratia* spp, *Klebsiella* spp, *Aeromosas* spp, and methods for preventing, treating and controlling mastitis in milk producing animals and for reducing its spread in dairy herds are described.

## Description

The present invention concerns the use of compositions containing terpinen-4-ol for the treatment and prevention of mastitis in milk producing animals. Particularly, the invention relates to a teat dip and to an intramammary terpinen-4-ol based compositions and methods for its applications directed to the prevention, treatment and control of mastitis in milk producing animals and to the reduction of the spread of mastitis in dairy herds.

The teat canal is the route by which pathogens gain entrance to the mammary gland. This canal is sealed between milking, and during the dry period, by a keratin plug derived from the stratified epithelial lining of the canal. The teat end contains sphincter muscles that maintain tight closure between milking. After milking, about two hours are required for the sphincter to contract and close the teat canal inducing mechanical and circulatory impairments in teat tissues.

The milking is a critical operation in relation to the barrier efficacy of the teat canal. Milk flushes out the keratin plug, and the teat canal is distended by the vacuum and the milk flux. Usually after milking, when the teat canal is till relaxed or dilated from the vacuum applied by the milking machine, residues of milk remains on the tip of the teat, which can act as a medium for bacterial growth. Then, the bacteria can migrate through the teat orifice and into the internal teat cistern causing inflammation and blockages. It was shown that in many cases bacteria are present in the teat canal for weeks before causing intramammary infections, despite regular teat dipping. The mastitis causing bacteria can be spread among cows by contaminated milking units or machines, by cow to cow contact, or can be transferred by milking personnel having contaminated hands. General housing conditions, such as stall size, ventilation, bedding material, and access to pasture are also known to have an impact on mastitis spread.

Often during milking, the skin of the dairy animal is irritated by automatic milking machines. This irritation, characterized by redness and occasionally areas of broken skin, can be the site of a microbial attack causing mastitis. Improper machine milking use or maintenance favours teat end erosion, and in the long term is likely to alter the functioning of the teat sphincter. Milking machine may also modify the immune defences of the teat duct.

Mastitis is a disease typically caused by microbial intramammary infection (IMI) that induces inflammation of the mammary gland. This pathology that essentially affect all lactating mammals, is especially problematic for dairy cattle and milk producing small ruminants. Animal mastitis, both clinical and sub clinical infections, results to be the disease causing the biggest economic losses to the dairy industry of developed countries, despite the intensive research and prevention measures at herd level carried out for decades. Its impact is on animal production, animal health and welfare and the quality of the produced milk. In Europe, the EEC regulation 853/2004 stated that milk with a somatic cell count (SCC) over 400.000 cells per mL may not be used for fluid milk and not even for human consumption. Also, human health concerns may be raised by milk consumption because of antibiotic residues in milk, transfer of antibiotic resistance from animal to human *via* zoonotic bacteria, and transfer of pathogens or products thereof through milk or milk products.

Mastitis is prevalently caused by bacteria, such as *S.aureus, St.agalactiae, St uberis* but also different species of streptococci, coliforms and *Aeromonas*, *Klebsiella* which enter the teat through the teat orifice. Contagious mastitis is spread during the milking process through contact between the animal and dairy equipment that may carry a source of a mastitis pathogen. A second type of mastitis, environmental mastitis, is caused by contamination of the animal surface by materials from the barn yard environment, fields, barn interior, etc. Such contamination occurs as the animal moves through its environment.

As a result, a significant amount of attention has been focused on preventing the development of mastitis or treating mastitis in dairy herds. Several control programs have been designed after the recognition of the multifactorial nature of mastitis. Proper milking procedures, biosecurity to prevent introduction of pathogens, segregation or culling of chronically infected animals and use of post-milking teat disinfectants are some basic issues of the mastitis control strategies.

Postmilking teat disinfection have long been used as a prevention of mastitis in animals during lactation and adopted by dairy producers in increasing numbers because it is recognized as a simple, economical, and highly effective method for reducing bacteria on teat skin and preventing the mastitis. The overall effectiveness of postmilking disinfection has been improved by the use of protective coatings on the animals. One class of coating compositions are the barrier dips which leave a germicidal film on the teats to help protect the teats during the milking and intermilking periods. Barrier dips are designed to effectively reduce infection caused by environmental bacteria as well as reducing the spread of infections caused by contagious bacteria. Teat dips can function by providing a physical barrier to bacterial entry through the teat orifice. Moreover, bacteria that may be present can be killed by antibacterial ingredients of some teat dips. Earlier versions of barrier dips produced a latex-type film that had to be peeled or washed off from the teats before milking. More recent formulations of barrier dips contain film-forming ingredients that remain semimoist on the teat surface. Post milking teat dipping is to day considered to be the single most important factor in mastitis prevention. The efficacy of teat dipping may be enhanced by the use of dry off therapy to reduce duration of existing infections.

Typically, the procedure of applying teat dip includes filling a cup or others suitable containers with the dip formulation followed by dipping the teat. An aerosol spray generally may include the same (or slightly modified) composition of the liquid used and is sprayed on to the teat. The aerosol spray generally works in the same way as dips, except for that the aerosol can have a chilling effect on the teat, causing the contraction of the sphincter muscle and the teat orifice, providing an additional obstacle to prevent bacterial entry.

A wide variety of germicides as chlorine, iodine (in particular iodophors), chiorhexidines, quaternary ammonium compounds, organic chloramines, bronopol and dodecyl benzene sulfonic acid (DDBSA) have been studied extensively as disinfectants for teat dips because it has been shown they have a wide spectrum of antibacterial activity against both Gram-positive and Gram-negative pathogens.

When disease prevention in dairy cattle is unsuccessful, antimicrobial drugs are used to treat certain conditions to maintain animal welfare and restore health. The treatment of intramammary infections concerns antimicrobial substances. A wide variety of antibiotics have been studied extensively for intramammary infusion because it has been shown they have a wide spectrum of antibacterial activity against both Gram-positive and Gram-negative pathogens. Therapeutic strategies involve administration of immediate release antibiotic formulations with or without long-acting formulations during the non-lactating periods, known as dry-cow therapy (DCT), or antibiotic treatment at end of lactation, is used to eliminate intramammary infections and prevent new infections during the dry period, even during lactating periods. It is one part of a total management system recommended in controlling intramammary infections in the milk producing animals.

When such products are used during lactation it is important that the antibacterial agent is eliminated as quickly as possible after the treatment has been effected. In this way a minimum of milk is wasted before the level of antibacterial agent in the milk has been reduced to a level acceptable to the health organisations for human consumption.

Human health concerns advise prudent use of antimicrobial drug therapy, as their use may leave antibiotic residues in the food chain, promote bacterial antibiotic resistance, transfer of antibiotic resistance from animal to human through zoonotic bacteria, and transfer of pathogens or products thereof through milk or milk products. Antimicrobial drug use exerts selective pressure on bacteria and eventually results in the development of antimicrobial resistance by some strains of bacteria. Depending on the mechanism of resistance, it may be passed to or amplified by successive generations of bacteria, or possibly exchanged among different strains of bacteria. This may result in pathogens of animals becoming resistant to certain antimicrobial drugs or classes of drugs. More likely, transfer could occur in nonpathogenic bacteria that might be consumed in improperly handled or prepared food, which in turn pass resistance components to human pathogens.

Some herds that practice a mastitis control program that includes a postmilking teat dip and a *dry cow therapy* (DCT) still have problems in controlling mastitis. Some reports have indicated that the failure of mastitis control programs can be at least partly attributed to the teat disinfectants and/or antibiotics which do not afford equal protection against the numerous pathogens that cause mastitis especially towards *Staphylococcus aureus* which is responsible for chronic infections and huge economic losses or environmental pathogens such as coliforms and streptococci other than *Streptococcus agalactiae.*

In the light of the above, it is evident the need of new methods and compounds for the treatment of mastitis in milk producing animals which allow to overcome the disadvantages of the known methods adopted so far.

There is a considerable and growing international literature on the use of essential oils and their components distilled from vegetable materials in human and veterinary medicine because of their potential to express antimicrobial activities. In some respects, this has been motivated by continuing resistance developed by infective organisms and diseases to conventional therapies. Literature data indicate that terpinen-4-ol is a component of essential oils derived from species of genera *Melaleuca, Leptospermum Origanum, Cupressus, Chamaecyparis, Juniperus, Elettaria, Myristica, Thymus* and mostly responsible for the efficacy in treating of many human infections. However, there is still a paucity of experimental data confirming and strengthening the activity of terpinen-4-ol against commensal skin or pathogenic microorganisms from animals.

The Authors of the present invention have now found that terpinen-4-ol can be advantageously used for preventing, treating and controlling mastitis in milk producing animals and for reducing its spread in dairy herds. Particularly, it has been found that terpinen-4-ol is efficacious against a wide range of mastitis causing bacteria of the genera *Staphylococcus* spp, *Streptococcus* spp, *Escherichia* sppp, *Enterococcus* spp, *Serratia* spp, *Klebsiella* spp, *Aeromosas* spp.

The terpinen-4-ol (trade names/synonyms: (+/-)-4- terpineol; (+/-)-p-menth-1-en-4-ol; (+/-)-para-menth-l-en-4-ol; (+/-)-terpinen-4-ol; 1-terpinen-4-ol; 1-para-menthen-4-ol; 3-cyclohexen-l-ol, 4-methyl-1-(1-methylethyl)-; 4-carvomenthenol; 4-carvomenthenol, natural; 4-methyl-1-(1-methylethyl)-3-cyclohexen-1-ol; 4-methyl-1-isopropyl-3-cyclohexen-1-ol; 4-terpinenol; 4-terpineol; dl-4-terpineol; menth-1-en-4-ol; para-menth-1-en-4-ol; p-menth-1-en-4-ol; terpinenol-4; C₁₀H₁₈0; 00203568; RTECS OT0175110 ; CAS NUMBER: 562-74-3; EC NUMBER (EINECS): 209-235-5) used according to the invention is a liquid, colourless to yellow, odor characteristic - mild earthy green with slight wood/pepper aspects, very soluble in alcohol and ether, fairly soluble in water (<0.5%). Terpinen-4-ol is available commercially from the producers Acros Organics BVBA, Geel, Belgium, catalogue number 360020000, 360020250, 36002100097%, from Hangzhou Standard Chemical Co., Ltd. China, from Sanmenxia Xiawei Chemical Co., Ltd. China and from Takasago International Corporation, Japan 2906.14.0000 C020007. Terpinen-4-ol may be produced by synthesis or extracted by fractional distillation of essential oil of Melaleuca alternifolia Cheel (Melaleucol™, from BGR CORPORATION PTY LTD., Level 6, 130 Phillip Street Sydney WALES 2000) or other species of the genus Melaleuca (Myrtaceae), or *Leptospermum*, *Origanum, Cupressus, Chamaecyparis, Juniperus, Elettaria, Myristica, Thymus*, but heretofore, it has not been used as a bactericide nor fungicide.

The antimicrobial activity of terpinen-4-ol is described in the literature but never it was used against bacteria, yeast, moulds and parasites isolates from animals and/or to control the mastitis.

Papadopoulos *et al*. tested for susceptibility 30 environmental or clinical isolates of *Pseudomonas* spp. to *Melaleuca altemifolia* (tea tree) oil and some of its components (including the terpinen-4-ol) founding no difference in the susceptibility of the soil and clinical isolates as was discussed in J Antimicrob Chemother (2006;_58(2):449-51) but he never studied the antibacterial activity of terpine-4-ol against mastitis isolates of *Pseudomonas* spp. Cox *et al*. compared the antimicrobial activity of *Melaleuca altemifolia* (tea tree) oil with that of some of its components, both individually and in two-component combinations on *E.coli, S.aureus, Candida albicans, Ps aeruginosa* culture collection finding the probable explanation of in vitro reduction activity of TTO as was outlined in J Appl Microbiol (2001;91(3):492-7 ) but they never studied the activity of terpine-4-ol against of microbial agents of mastitis. Hammer *et al*., investigated the mechanism of action of tea tree oil and its components on *Candida albicans, Candida glabrata* and *Saccharomyces cerevisiae* as was reported in J Antimicrobial Chemotherap (2004;53(6):1081-5). They demonstrated that terpinen-4-ol is one of the most active components of TTO exerting their antifungal actions by altering membrane properties and compromising membrane-associated functions but they never demonstrated antimicrobial activity against mastitis pathogens. Carson CF and Riley TV studied the antimicrobial activity of eigth individual components of tea tree oil (TTO) including terpinen-4-ol on the following microrganisms : *Bacillus subtilis* NCTC 3610, *Bacteroides fragilis* NCTC 9343, Candida albicans ATCC 10231, *Clostridium perfringens* NCTC 8359, *Enterococcus faecalis* NCTC 8213, *Escherichia coli* NCTC 10418, *Lactobacillus acidophilus* NCTC 2949, *Moraxella catarrhalis* NCTC 3622, *Mycobacteium smegmatis* NCTC 333, *Pseudomonas aeruginosa* NCTC 10622, *Serratia marcescens* NCTC 1377 and *S.aureus* NCTC 6571 as was reported in J Appl Bacterol (1995; 78:264-269) but they not studied the activity on wild pathogens isolated from animals diseases.

The complete mechanism of action by which it acts on microrganisms is unknown, but it is thought that terpinen-4-ol kills microrganisms by lysing the cellular membrane causing a lethal damage of the cells of the organisms. In practice, teat, on which it is desirable to kill or prevent the growth of microrganisms, is treated with bactericidal concentrations of terpinen-4-ol by dipping incorporating it in gels and sprays.

The production of terpinen-4-ol containing preparations having long storage stability is exceptionally demanding. With the preparation according to the invention it has been possible to achieve this characteristic because of stability at normal temperatures and pressure. Thus, the preparation according to the invention has exceptionally high storage stability because of the intrinsic strong wide spectrum antimicrobial activity of terpinen-4-ol to prevent microbial spolilage and the inertia of the gelling agent.

Therefore, it is specific object of the present invention a veterinarian composition comprising or consisting of terpinen-4-ol as active compound in combination with one or more excipients and/or adjiuvants pharmaceutically acceptable for use in the prevention and treatment of mastitis in milk producing animals. Preferably, terpinen-4-ol is used in a concentration from 1 to 10 %, over the total weight of the composition, more preferably 1-2%. According to the invention, terpinen-4-ol can be synthetic or a natural one. Particularly, the composition of the present invention is efficacious in the treatment and prevention of mastitis caused by bacteria of the genera, *inter alia*, *Staphylococcus* spp, *Streptococcus* spp, *Escherichia* sppp, *Enterococcus* spp, *Serratia* spp, *Klebsiella* spp, *Aeromosas* spp.

The compositions can comprise a 0,1-10 % by weight of terpinen-4-ol; about 0,2 to 5% of a gelling agent; an amount of a pH adjusting agent sufficient to adjust the final pH from about 5 to less than 6; and water in an amount sufficient to make up the balance of the composition. Vanillic acid and/or cinnamic aldehyde in the range of 0,1-5% can be added. The compositions can be applied to the teat either as a "dip", directly from a cup or similar container, or can be applied as a spray or by plastic syringe for intramammary infusion via the teat canal. If it is desired that the composition is applied as an aerosol spray, a propellant such as dimethyl ether or propan, buthan, CO₂, N which can also function as a carrier, can be included. A composition of the present invention can also be applied by non-aerosol spray methods such as trigger or pump sprays.

According to another aspect of the present invention there is provided an antimicrobial composition, substantially as described above, which does not create complication on animal health because of terpinen-4-ol decrease the skin integrity dose-dependently and ,consequently, is very well tolerated. Accordingly, it is an object of the present invention a preparation combining the terpinen 4-ol with one or more extracts chosen from the group consisting of *Anthemis nobilis (Matricaria recutita*) extract, *Agrimonia eupatoria* extract and *Calendula officinalis* extract preferably in a range of about 0.5% to 10% and/or lactoferrin in the range of about 1000 and 4000 mcg/g or ml.

Generally, the majority of the composition, or "carrier", is a liquid. The carrier may or may not act as a solvent for solubilizing the active ingredients of the composition. Usable liquid carriers include water (including tap water, distilled water, deionized water), propylene glycol and alcohol, preferably the lower alkanols of 3 carbon atoms or less, such as propanol, ethyl alcohol and methyl alcohol. The carrier is generally the largest portion of the formulation, typically present in a range of about 40% to 90% by weight. Alcohol and glycol, if present, are typically in a range up to about 5-10%

The gelling agent can be used in the amount of up to about 5 %, preferably from about 0.4 to 1.0 %, and more preferably about 0.7 %. It is selected from the group consisting of carboxyvinyl polymers, hydroxycellulose, hydroxyethyl cellulose, hydroxypropylmethyl cellulose, methylcellulose, carboxymethylcellulose, hydroxypropyl cellulose, and alginic acid-propylene glycol ester. Examples of suitable gelling agents include carboxyvinyl polymers, hydroxycellulose, hydroxyethyl cellulose ("HEC"), methylcellulose, hydroxypropylmethyl cellulose ("HPMC"), carboxymethyl cellulose, hydroxypropyl cellulose ("HPC"), alginic acid-propylene glycol ester, or polyacrylic acid polymer ("PAA"). Preferred gelling agents are HPC, available from Hercules, Inc. as KLUCEL HF, and PAA, available from B. F. Goodrich Chemical Co. as CARBOPOL, CARBOMER 934 NF, or CARBOMER 980 NF. Other gelling agents may be alternatively used in this invention provided they are compatible with the hydroalcoholic system, i.e., capable of forming a gel with the amount of alcohol and water required to solubilize terpinen-4-ol.

The pH of the composition may be adjusted to the desirable range by the addition of a pH adjusting agent, such as, for example, NaOH, diisopropanolamine, diisopropylamine, triethanolamine, diethylamine, triethylamine, sodium hydroxide, potassium hydroxide or any other compatible, pharmaceutically acceptable base or alkalizing agent. These neutralizing agents may preferably be used in an amount by weight of up to about 10.%. The optimal value of the formula in the range of 5.5-6.0

Preferably, the hydroalcoholic gels for use in the process of the present invention will have a viscosity of within the range of about 3000 to 10.000 centipoises (cps), preferably when measured using a viscometer with helipath stand at room temperature (i.e., 20°-25°C.) and with a 0.5 inch helipath and T-spindle (size "E") rotating at 0.3 RPM in a sample size ranging from 15 to 20 grams. However, even a broader range of viscosity is possible.

The composition for teat dip may also include additional additives, such as pigment or dye, to act as an indicator whether a particular cow has been treated with the composition. Typically, a dye, for example FD & C Blue Dye #3 or F D & C Green color #3 is included in a range of about 0.01% to 1.0 %, preferably about 0.05% of the total composition.

It is a further object of the present invention for the prevention and treatment of mastitis, the use of vegetable and/or mineral oils as vehicle of the active principles in the composition of intramammary infusion formula for dry-off treatment . The final composition is in the range 0,5 to 2% in oil.

Finally, it is an object of the present invention the terpinen-4-ol compound for use in the prevention and treatment of mastitis in milk producing animals. Mastitis to be treated are caused by bacteria of the genera, *inter alia*, *Staphylococcus* spp, *Streptococcus* spp, *Escherichia* sppp, *Enterococcus* spp, *Serratia* spp, *Klebsiella* spp, *Aeromosas* spp.

Basic aspects of the present invention will become apparent from the following Tables and Figures that are given by way of examples only. Tables and Figures summarize the principal experiments conducted to determine the antimicrobial efficacy of the terpinen-4-ol and its potential application.

The present invention will now be described in reference to exemplifying but not limiting preferred embodiments thereof with particular reference to the figures of the enclosed drawings wherein:

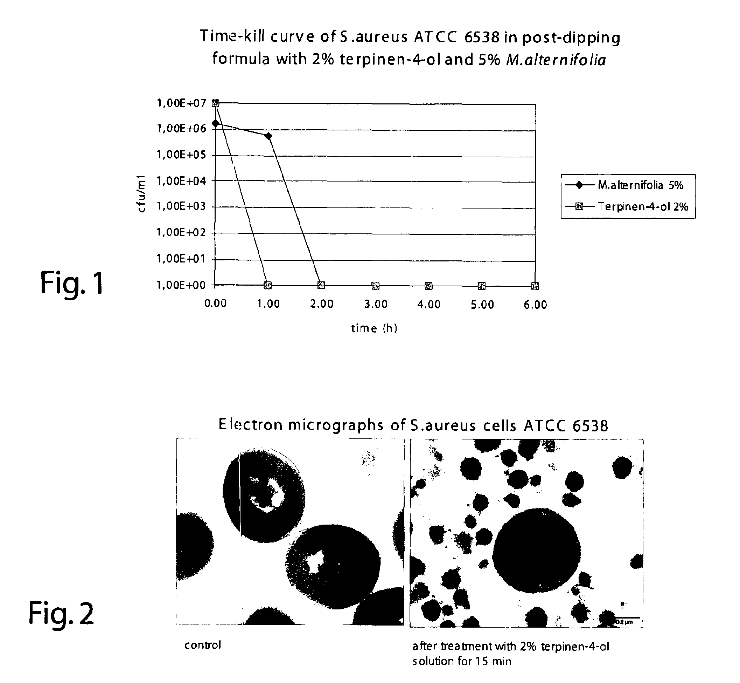

Figure 1 shows the time-kill curve of S. aureus ATCC 6538 in post-dipping formula with 2% terpinen-4-ol and 5% M. alternifolia. Killing assay evaluates the reduction in the numbers of cfu/ml over 2 h. Post-dipping formula were inoculated in standardized conditions with S.aureus. No viable cells were detected in post-dipping formula with terpinen-4-ol after 1 hr.
Figure 2 shows electron micrographs of *S*. *aureus* cells ATCC 6538 for control group and after treatment with 2% terpinen-4-ol solution for 15 min.

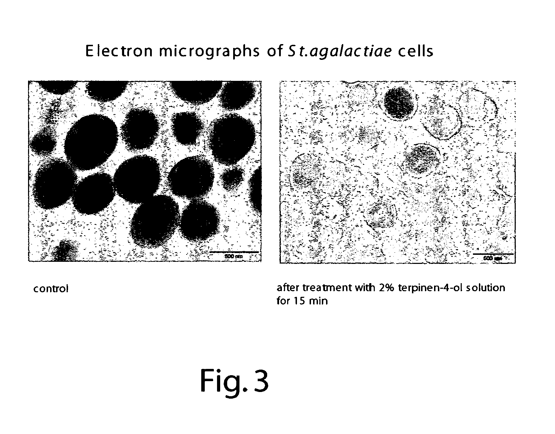

Figure 3 shows electron micrographs of *St. agalactiae* cells for control group and after treatment with 2% terpinen-4-ol solution for 15 min.

Strain suspensions of *S.aureus* ATCC 6538 and a wild strain of *St.agalactiae* cultured in appropriate nutrient media were standardized by spectrophotometer at 625 nm to a O.D. of about 3.0 and subsequently exposed to terpinen-4-ol at the concentration of 0.2% and respectively to *M. alternifolia* e.o. at the concentration of 5% for 10 min. After centrifugation the pellets were treated as described by Carson, 2002 [Carson SF et al. Mechanism of Action of Melaleuca alternifolia (Tea Tree) Oil on Staphylococcus aureus Determined by Time-Kill, Lysis, Leakage, and Salt Tolerance Assays and Electron Microscopy Antimicrob Agents Chemother. 2002 June; 46(6): 1914-1920].

Electron microscopy of terpinen-4-ol and *M.alternifolia* e.o. treated cells showed the formation of mesosomes and the loss of cytoplasmic contents. The predisposition to lysis, the loss of 260-nm-absorbing material, the loss of tolerance to NaCl, and the altered morphology seen by electron microscopy all suggest that both tea tree oil and terpinen-4-ol compromise the cytoplasmic membrane.

### EXAMPLE 1: Study on bactericidal activity of 2% terpinen-4-ol solution

Table 1 shows the bactericidal activity of 2% terpinen-4-ol solution tested by UNI/EN1656 on 230 mastigenic wild strains (r = Log colture broth reduction after 30 min of exposure)

UNI/EN 1656 (UNI EN 1656:2000 - Chemical disinfectants and antiseptics - Quantitative suspension test for the evaluation of bactericidal activity of chemical disinfectants and antiseptics used in veterinary field - Test method and requirements) is a quantitative suspension test for the evaluation of bactericidal activity of chemical disinfectants and antiseptics used in veterinary field.

A standardized test suspension of each bacterium in a solution of an appropriate interfering substance is added to a prepared sample of the product under test. The mixture is maintained at 30°C + 1°C. At a contact time of 30 min an aliquot is taken; the bactericidal action in this portion is immediately neutralized by an appropriate neutralizer.

The number of surviving bacteria in each sample is determined and the logarithmic reduction in viable counts calculated and reported as r.

**Table 1**

| **Species** | **No.strains** | **r ≥ 10³** | **r ≥ 10⁴** | **r ≥ 10⁵** |
|---|---|---|---|---|
| *S. aureus** | 88 | 100% | 98% | 86% |
| *Staphylococcus spp.* | 19 | 95% | 79% | 47% |
| *St agalactiae* | 27 | 89% | 89% | 67% |
| *Streptococcus spp.* | 46 | 98% | 96% | 87% |
| *E. coli* | 14 | 93% | 93% | 93% |
| *Enterococcus spp.* | 21 | 95% | 95% | 90% |
| *S. marcescens* | 7 | 100% | 100% | 100% |
| *Kl. Pneumoniae* | 2 | 100% | 100% | 100% |
| *Klebsiella spp.* | 3 | 33% | 33% | 33% |
| *A. viridans* | 3 | 100% | 100% | 100% |

| | | | | |
|---|---|---|---|---|
| * 64/77 (83%) of the strains of *S.aureus* showed antibiotic resistance to antibiotics commonly used in vet field. | | | | |

Table 2 shows the bactericidal activity of 2% terpinen-4-ol solution tested by UNI/EN1656 on 62 wild mastitogenic multiple antibiotic resistant *S.aureus* strains

**Table 2**

| **n° strains** | **antibiotic resistance patterns** | **% strains exhibiting r>5*** |
|---|---|---|
| | PenG-Oxa-Cef-Tc-Tmp/Smx | |
| | PenG-Oxa-Cef-K-Tmp | |
| 3 | Smx; PenG-Tc-K-Enr-Tmp/Smx | 100 |
| | PenG-Tc-K-Tmp/Smx | |
| 3 | PenG-Tc-Enr-Tmp/Smx | 100 |
| | PenG-Tc-Tmp/Smx | |
| 8 | Tc-K-Tmp/Smx | 100 |
| | PenG-Tmp/Smx | |
| | Tc-Tmp/Smx | |
| | Oxa-Tmp/Smx | |
| 22 | K-Tmp/Smx | 91 |
| 26 | Tmp/Smx | 85 |
| **tot 62** | | |

| | | |
|---|---|---|
| * r= Log reduction target of culture test to prove the efficacy of test solution used as antiseptics in veterinary field Molecules tested : PenG : Penicillin G; Oxa: Oxacillin; Cef: Cefazolin; Tc: tetracycline; Tmp/Smx: Trimethoprim-sulfamethoxazole; K: kanamycin; Enr:enrofloxacin. | | |

The concentration of 2% of terpinen-4-ol proved effective on bacterial wilde mastitogenic strains of *S.aureus* showing antibiotic resistance up to 5 differents molecules. All the multiantibiotic-resistant strains resulted sensitive to terpinen-4-ol, stressing the potential advantage of terpinen-4-ol as alternative treatment against antibiotic resistant bacteria

Table 3 shows the challenge test to evaluate the stability of the post-dipping preparation containing 2% terpinen-4-ol

**Table 3**

| **microrganism** | **Initial inoculum /g** | **1-5 months** CFU/ g | **Month 7** |
|---|---|---|---|
| *S.aureus* | 5x 10⁷ | < 10 | 4.5 x 10² |

7 months after the preparation the formula remains effective showing a reduction of 7 log when inoculated with a standardized inoculum of S.aureus.

Table 4 shows the detection of terpinen-4-ol residue in milk by HS-GC/MS-SIM after post dipping treatment

The terpinen-4-ol residue was identified and quantified by HS-GC/MS-SIM. The capillary column was an HP-5MS column (25 mm x 0.25 mm i.d. x 0.25 microm); oven temperature increased with a rate of 5 degrees C /min from 80 to 300 degrees C, and then maintained for 20 min; sample size of 1 microL; split ratio of 100:1; carrier gas of helium (1 mL/min). Mass spectra were obtained at 70 eV. The temperatures of injector base and ionization source were maintained at 270 °C and 230 °C, respectively. The test performed on sample collected as in a worst case demonstrated the suitability of the method for testing incurred samples. This method, applied to milk samples collected during the experimentation for teat post-milking dipping, allowed to detect residues of terpinen-4-ol at a LOD of 0.1 mcg/L. The quantification of the terpinen-4-ol residue evaluated a concentration equivalent to 0.2 mcg/L

**Table 4**

| **Samples** | **concentration** | **LOD** |
|---|---|---|
| Milk from post dipping treatment * | 0.2 mcg/L | |
| Control milk | neg | |
| | | 0.1 mcg/L |

| | | |
|---|---|---|
| *: collected after 12 hrs without throwing away the first squirt of milk as recommended in GMP | | |

Table 5 shows the minimum residue concentration of terpinen-4-ol detectable by microbial screening for inhibitors (Delvo test) in milk

The Regulation EC 853/2004 charges food business operators producing or collecting raw milk with a task to ensure that milk not come from animals to which unauthorised substances or products have been administered or, where authorised products or substances have been administered, to observe the withdrawal periods prescribed for these products or substances. The continuous monitoring control of residues of veterinary medicinal products in milk is under Regulation 882/2004/CE and specifically, the control of residues of antibiotics is routinely performed through a microbial inhibitor screening test. The screening is based on an agar diffusion test in a medium inoculated with spores of *B. stearothermophilus* var. *calidolactis*. The presence in the milk of substances inhibitory to the growth of the microorganism test is detected in about 2.30 h. To investigate the possibility that the residue of terpinen-4-ol could interfere with this test focused to the research of antibiotics, has been evaluated the minimum residue concentration of terpinen-4-ol giving a positive reaction to this method. For this trial, a commercial kit .....was used. Results obtained showed that terpinen-4-ol concentration needed to produce a positive result in a microbial screening test is 2500 mg/L, meaning an unattainable concentration in the normal condition of teat post-milking dipping application

**Table 5**

| **Samples** | **Results** |
|---|---|
| Milk from post dipping treatment * | negative |
| Milk spiked with terpinen-4-ol : 2500 mg/L | positive |
| Control milk | negative |

| | |
|---|---|
| *: collected after 12 hrs without throwing away the first squirt of milk as recommended in GMP | |

The presence of residues of terpinen-4-ol gives no positive reaction to the routine microbial screening test for the research of inhibitors in milk, as the residue is well below the detection limits of the test.The continuous monitoring control of residues of veterinary medicinal products in milk is under Regulation 882/2004/CE (EC 2004).

## Claims

1. Veterinarian composition comprising or consisting of terpinen-4-ol as active compound in combination with one or more excipients and/or adjiuvants pharmaceutically acceptable for use in the prevention and treatment of mastitis in milk producing animals.

2. Composition according to claim 1 wherein terpinen-4-ol is in a concentration from 1 to 10 % over the total weight of the composition, preferably 1-2%.

3. Composition according to anyone of the previous claims further comprising one or more extracts chosen from the group consisting of *Anthemis nobilis* extract, *Agrimonia eupatoria* extract or *Calendula officinalis* extract.

4. Composition according to claim 3, wherein the concentration of said extracts ranges from 0.5% to 10% over the total weight of the composition.

5. Composition according to anyone of the previous claims, further comprising lactoferrin.

6. Composition according to claim 5, wherein lactoferrin is in the range of about 1000 and 4000 mcg/g or ml.

7. Composition according to anyone of the previous claims comprising terpinen-4-ol 0,1-10% by weight, a gelling agent 0,2-5% by weight, a pH adjusting agent in order to have a pH from 5 to 6.

8. Composition according to anyone of the previous claims, further comprising vanillic acid and/or cinnamic aldehyde.

9. Composition according ot claim 8, wherein vanillic acid and/or cinnamic aldehyde are in the range 0,1-5% over the total weight of the composition.

10. Composition according to anyone of the previous claims wherein mastitis are caused by bacteria, of the genera *Staphylococcus* spp, *Streptococcus* spp, *Escherichia* sppp, *Enterococcus* spp, *Serratia* spp, *Klebsiella* spp, *Aeromosas* spp.

11. Composition according to anyone of the previous claims, comprising vegetable and/or mineral oils as vehicle of the active principle.

12. Composition according to claim 11, wherein the concentration of the oil is from 0,5 to 2% over the total weight of the composition.

13. Terpinen-4-ol compound for use in the prevention and treatment of mastitis in milk producing animals.

14. Terpinen-4-ol compound for use according to claim 13, wherein mastitis are caused by bacteria of the genera *Staphylococcus* spp, *Streptococcus* spp, *Escherichia* sppp, *Enterococcus* spp, *Serratia* spp, *Klebsiella* spp, *Aeromosas* spp.
